# EUROPÄISCHE PATENTSCHRIFT

(11) **EP 3 287 074 B1**
(45) Veröffentlichungstag und Bekanntmachung des Hinweises auf die Patenterteilung: **26.12.2018**
(21) Anmeldenummer: 16185273.6
(22) Anmeldetag: 23.08.2016
(51) Int. Cl.: A61B 5/16, A61B 5/00

(54) **VERFAHREN ZUR ERFASSUNG DER ZEREBRALEN KOGNITIONSZEIT SOWIE VORRICHTUNG ZUR ERFASSUNG DER ZEREBRALEN KOGNITIONSZEIT**
METHOD FOR DETECTING THE CEREBRAL COGNITION TIME AND DEVICE FOR MONITORING OF CEREBRAL COGNITION TIME
PROCEDE DE DETECTION DU POUVOIR DE COGNITION CEREBRALE ET DISPOSITIF ASSOCIE

(43) Veröffentlichungstag der Anmeldung: 28.02.2018
(73) Patentinhaber: Talkingeyes&more GmbH, 91052 Erlangen (DE)
(72) Erfinder: Michelson, Georg, 91083 Baiersorf (DE)
(74) Vertreter: Stippl, Hubert

(56) Entgegenhaltungen:
- EP-A2- 0 251 541
- WO-A2-2007/135441
- CA-A1- 2 720 892

## Beschreibung

### Technologischer Hintergrund

Zur Erfassung von optischen oder akustischen Reizen, Gerüchen oder mechanischen Reizen besitzt der menschliche Organismus Sinnesorgane, die als Sensoren für die jeweiligen Reize dienen. Beispielsweise dient bei optischen Reizen das Auge zur Bildaufnahme, wobei jedoch die Mustererkennung, d.h. die Weiterverarbeitung bzw. Interpretation der aufgenommenen Information in der Sehbahn und im visuellen Cortex erfolgt. Die sensorische Fähigkeit wird charakterisiert anhand der sensorischen Schwelle (wie gering ist der Reiz, der gerade noch erkannt wird) und der Zeitdauer zur Erkennung des Reizes (wie schnell kann der Reiz detektiert werden). Die Schnelligkeit der motorischen Antwort auf einen detektierten und erfassten Reiz ist unabhängig von der sensorischen Schwelle und der Zeitdauer zur Erkennung des Reizes. Optische Reize können damit bis zu einer bestimmten Schwelle hinsichtlich der Ortsauflösung, des Kontrastes, der Texturstärke und der Stereodisparität erkannt werden. Diese sensorische Schwelle ist abhängig von vielen individuellen Faktoren und zeigt sich vermindert bei bestimmten Erkrankungen (Photorezeptorenschädigung, Linsentrübung, etc.). Die Zeitdauer der zerebralen Verarbeitung bzw. des Erkennens der von dem Auge aufgenommenen Bilddaten ist abhängig vom "Schwierigkeitsgrad" des optischen Reizes. Die Zeitdauer der Verarbeitung bzw. des Erkennens der dargebotenen visuellen Stimuli im zerebralen Anteil der Sehbahn zur Interpretation der Bilddaten wird "zerebrale Kognitionszeit" genannt. Die zerebrale Kognitionszeit kann von Patient zu Patient stark variieren. Beispielsweise haben Patienten mit bestimmten Krankheitsbildern (z.B. Patienten, die an MS erkrankt sind) längere zerebrale Kognitionszeiten als gesunde Menschen. Die zerebrale Kognitionszeit kann auch durch Ermüdungs- oder Erschöpfungszustände, Alkoholeinfluss, Medikamenteneinfluss beeinträchtigt sein. Auch ist es möglich, dass sich die zerebrale Kognitionszeit durch häufiges Wiederholen der gleichen Aufgabe bei individuellen Testpersonen nach und nach verbessert. Um gesicherte Diagnoseergebnisse zu erhalten, besteht daher ein großes klinisches Bedürfnis dafür, die zerebrale Kognitionszeit so genau wie möglich zu erfassen und von der motorischen Reaktionszeit abzugrenzen. Die motorische Reaktionszeit ist die Zeit zwischen erfolgreichem Abschluss des zerebralen Erkennungsprozesses und Abschluss der motorischen Reaktion (z.B. Tastendruck). Die motorische Reaktionszeit ist unabhängig von der zerebralen Kognitionszeit, aber stark abhängig von individuellen Faktoren (Geschicklichkeit, Übung, etc.) und vom Gesundheitsstatus der motorischen Nerven und Muskeln.

Interne Untersuchungen haben ergeben, dass um die zerebrale Kognitionszeit im Rahmen einer Diagnose oder Therapie feststellen zu können, Stimuli in Form von statischen oder dynamischen Bildern einer Testperson präsentiert und anhand der motorischen Reaktionen der Testperson darauf bestimmt werden können. Dabei werden die Stimuli als z.B. optische Reize überhalb und unterhalb der Erkennens-Schwelle präsentiert. Die optischen Bilder bieten der Testperson zwei oder mehr Wahloptionen, von denen nur eine korrekt ist und nur erkannt werden kann, wenn der Stimulus richtig erkannt wird. Mit der richtigen Wahl durch die Testperson wird ein korrektes Erkennen des Stimulus dokumentiert. In der Regel erfolgt die Wahl einer bestimmten Optionen auf den jeweiligen Stimulus durch eine motorischen Aktion, beispielsweise durch Betätigung eines bestimmten Tasters, in der Vornahme einer bestimmten Geste, im Drücken eines Fußschalters, durch ein akustisches Signal, usw.. Die motorische Reaktion zeigt dann, ob (1) der visuelle Stimulus korrekt erkannt wurde und (2) wie lang die Zeitdauer von Präsentation des optischen Stimulus bis Abschluss der motorischen Antwort war (Reaktionszeit). Hierbei wird als Maß für die Reaktionszeit das Zeitintervall beginnend vom Zeitpunkt der Präsentation des Stimulus an bis beispielsweise zur Betätigung des Tasters herangezogen. Neben der Reaktionszeit für unterschiedlich schwer zu erkennende optische Stimuli kann durch Präsentation ober- und unterschwelliger Stimuli die sensorische Schwelle ermittelt werden. Die sensorische Schwelle ist derjenige Schwierigkeitsgrad, bis zu welchem Schwierigkeitsgrad der Stimulus korrekt erkannt wurde. Auf diese Weise kann ein Schwellwert für das sensorische Erkennen ermittelt werden. Der klinische Wert der Reaktionszeitbestimmung ist beschränkt, da die Reaktionszeiten als Folge der Stimuli-Präsentation sich nicht lediglich auf die zerebrale Kognition beziehen, sondern eine Vielzahl weiterer Umstände, wie z.B. die motorisch bedingte Reaktion mitumfassen. Die gemessene Reaktionszeit ist das Zeitinvall, das mindestens die zerebrale Kognition (das Erkennen) und die motorische Antwort umfasst.

### Druckschriftlicher Stand der Technik

Ein Verfahren gemäß dem Oberbegriff des neuen Anspruchs 1 ist aus der EP 0 251 541 A2 bekannt. Das Ziel dieses Verfahrens besteht darin, die reflexive neuromuskuläre Leistung eines Sportlers, z. B. Boxers, unter kontrollierten Bedingungen zu trainieren. Das Verfahren umfasst das Erzeugen eines optischen oder akustischen Stimulus, die Voreinstellung eines bestimmten Schwierigkeitsgrades, das Erfassen der Reaktionszeit und die Anzeige einer Bewertung der Reaktionszeit durch die Auswahl einer bestimmten von mehreren LEDs. Der Schwierigkeitsgrad wird vorab auf der Grundlage von vorherigen Auswahlangaben bezüglich Körpergröße, Schlagtyp, Schwierigkeitsgrad die Komponenten festgelegt und mit empirischen Daten ergänzt.

Die WO 2007/135441 A2 offenbart eine Vorrichtung zur Detektion und/oder Einstufung von Defiziten in der Neuroentwicklung von Kindern, wie z. B. AHD, ADHS oder DCD. Die Vorrichtung umfasst ein Display zur Ausgabe einer Aufgabe sowie einer Eingabeeinrichtung, mittels welcher der Nutzer als Antwort auf die Aufgabe reagieren kann. Der Nutzer kann eine Reihe von Aufgaben, die in ihrem Schwierigkeitsgrad als Funktion der Leistung des Nutzers sich ändern, durchschreiten. Ein Schwellwert dient dazu auszuloten, bis zu welchem Schwierigkeitsbereich die Aufgaben gestellt werden können.

Die EP 2 094 159 B1 beschreibt ein System sowie ein Verfahren zum Nachweis eines bestimmten kognitiv-emotionalen Zustands einer Person. Das Verfahren umfasst das Bereitstellen eines individuellen Reizes, das Vorsetzen einer optischen Anzeige des individuellen Reizes vor die Versuchsperson über eine besondere Zeitdauer, das Erfassen sowie die Auswertung von Reaktionszeiten auf die Präsentation des individuellen Reizes. Das Verfahren dient dazu, den emotionalen Zustand der Versuchsperson in Bezug auf unterschiedliche Produkte zu erforschen.

Die DE 10 2005 025 977 A1 offenbart eine Zeitmessvorrichtung und ein Zeitmessverfahren für neuronale Entscheidungsprozesse. Hierbei erfolgt eine erste Serie von Präsentationen tabellarisch gehaltener verschiedener Stimuli, wobei deren Reaktionszeiten in einer Reaktionszeittabelle gespeichert werden, sowie eine zweite Serie von Präsentationen der gleichen Stimuli bei einer jeweils bewertungsabhängig invers vorgegebener Bestätigungsart, wobei die den jeweiligen Stimuli zugehörig ermittelten weiteren Reaktionszeiten ebenfalls in der Reaktionszeittabelle entsprechend gekennzeichnet gespeichert werden. Weiterhin erfolgt eine differentielle Auswertung der Reaktionszeiten bezüglich der jeweils vorgegebenen und dazu ausgeführten Betätigungsarten zu den Stimuli. Die Ergebnisse mehrerer Probanden werden gemittelt und verdichtet. Hierbei wird der Stimulus zu einem Probandenidentikator jeweils seitlich versetzt und/oder sich annähernd oder entfernend und/oder wachsend oder schwindend auf dem Monitor präsentiert. Die Eingabevorrichtung zur Erzeugung der verschiedenen Meldungsarten ist so ausgestaltet ist, dass dabei der Bedienerabstand zu ihr praktisch konstant ist. Ziel dieser Erfindung ist es, durch die beschriebenen Maßnahmen die Streuung der Reaktionszeiten auf die Stimuli zu minimieren.

Aus der Zeitschrift Markman A.B. et al., "Constraining Theories of Embodied Cognition", Psychological Science 2005, Band 16, Seiten 6-10 ist eine Vorrichtung bekannt, bei der auf einem Monitor perspektivisch mittig ein Probandenidentifikator dargestellt wird und der Stimulator perspektivisch vor oder hinter diesem präsentiert wird und zur Reaktion darauf eine Bewegung eines Eingabe-Schwenkhebels zum Körper des Probanden hin oder von diesem weg vorgegeben ist.

Aus dem publizierten Zeitschriftenartikel "Paulus J. et al, Intended stereopsis evaluation of professional and amateur soccer players and subjects without soccer background, Frontiers and Psychology 2014, Band 5, Seiten 1-8" sind Computer-unterstützte Stereopsis-Tests und deren Auswertungen bekannt, bei der stationäre sowie bewegende Stimuli sowohl Profifußballern als auch Gelegenheitsfußballern ausgesetzt wurden. Hierbei hat sich herausgestellt, dass Profifußballer, was die Stereopsie anbelangt, nicht wesentlich besser abschneiden als Gelegenheitsfußballer.

### Aufgabe der vorliegenden Erfindung

Die Aufgabe der vorliegenden Erfindung besteht darin, ein Verfahren sowie eine Vorrichtung zur Verfügung zu stellen, mit der die zerebrale Kognitionszeit genauer erfasst werden kann.

### Lösung der Aufgabe

Die vorstehende Aufgabe wird in Bezug auf das erfindungsgemäße Verfahren durch die Merkmale des Anspruchs 1 sowie für die erfindungsgemäße Vorrichtung durch die Merkmale des Anspruchs 10 gelöst.

Zweckmäßige Ausgestaltungen des erfindungsgemäßen Verfahrens sowie der erfindungsgemäßen Vorrichtung werden in den Unteransprüchen beansprucht.

Das erfindungsgemäße Verfahren erlaubt es, die zerebrale Kognitionszeit K unterschiedlich erkennungstechnisch schwieriger Stimuli mit sehr hoher Genauigkeit zu erfassen. Mit dem erfindungsgemäßen Verfahren erhält man eine Messreihe von Reaktionszeiten T bei allmählich absteigendem Schwierigkeitsgrad S der kognitiven Aufgabe, wobei die Reihenfolge der präsentierten Schwierigkeitsgrade der Stimuli nicht von schwer nach leicht, sondern zufallsverteilt ist. Man reizt gleichsam die kognitiven Fähigkeiten der Testperson unter Zugrundelegung von Stimuli bei sich änderndem Schwierigkeitsgrad aus, bildet einen Kurvenverlauf mit Messwerten der Reaktionszeiten von Stimuli mit unterschiedlichen Schwierigkeitsgraden und leitet aus dem asymptotischen Verlauf der Kurve einen Korrekturwert der Reaktionszeit ab, um den die tatsächlich gemessene Reaktionszeit bei dem betreffenden Stimulus bereinigt werden kann. Hierdurch kann eine abgeleitete Kognitionszeit K(S) für jeden Schwierigkeitsgrad und damit auch eine abgeleitete Kognitionszeit für den sensorischen Schwellenwert (=K(Schwellwert)) festgelegt werden, die um die aus der Motorik resultierenden Zeitkomponenten bereinigt ist. Die Messwertkurve aus Reaktionszeiten für unterschiedliche Stimulusschwierigkeitsgrade entspricht, zumindest im Wesentlichen dem Verlauf einer Exponentialfunktion. Daraus resultiert der Vorteil, dass die individuellen, die motorische Reaktionszeit beeinflussenden Faktoren (wie z.B. händische Geschicklichkeit, bestimmte Krankheiten, tagesformabhängige Faktoren, medikamentös bedingte Faktoren usw.) bei der Feststellung der zerebralen Kognitionszeit wirksam ausgeschlossen werden können. Vor allem ist dies vorteilhaft, wenn im Zuge von Therapiemaßnahmen die abgeleitete Kognitionszeit für den Schwellwert K(Schwellwert) als Referenz- und/oder Steuervariable herangezogen wird. Hierdurch wird ein genaues Feedback z. B. im Rahmen einer Therapiemaßnahme und damit eine wesentlich gezieltere Therapie ermöglicht. Beispielsweise kann die Wirksamkeit eines Medikaments hierdurch wesentlich genauer überprüft werden. Ebenso ermöglicht die Erfindung eine genauere Kontrolle des Therapieeffekts z. B. im Rahmen von häufig durchgeführten Testreihen. Weiterhin kann die Fläche unter der Kurve zwischen dem Punkt T(Schwellwert)/ S(Schwellwert) und dem Punkt T(1)/ S(1) als weiteres klinisches Maß für die Kognitionsfähigkeit und den Krankheits- oder Therapieverlauf herangezogen werden.

Bei der unteren Reaktionszeit T1 handelt es sich um einen Zustand oder einen Bereich der motorischen Reaktionszeit, der unabhängig ist vom Schwierigkeitsgrad des präsentierten Stimulus ist oder der zumindest eine bestimmte Reaktionszeit-Bandbreite nicht verlässt, da er die motorische Reaktionszeit nach erfolgreichen Erkennens des Stimulus präsentiert.

Gemäß der Erfindung wird die abgeleitete Kognitionszeit des sensorischen Schwellwertes K(Schwellwert) oder eines Stimulus mit einem sensorischen Schwierigkeitswertes K(x) als Referenzvariable und/oder Steuervariable für ein Diagnose- und/oder Trainingsverfahren zur Diagnose zum Training der kognitiven Leistungsfähigkeit verwendet. Änderungen dieser abgeleiteten Kognitionszeit K(Schwellwert) oder K(x), beispielsweise im Rahmen eines Trainingsprogramms oder Therapieansatzes, zeigen daher unmittelbar eine Änderung der kognitiven Leistungsfähigkeit unabhängig von der motorischen Reaktionsfähigkeit an. Bei ausschließlicher Verbesserung der motorischen Reaktionsfähigkeit bleibt der Kurvenverlauf konstant, nur verschiebt sich die Kurve in der y-Achse.

Bei Verbesserung der Kognitionsfähigkeit kann die Kurve flacher werden und es kommt zu einer Verschiebung der Kurve in der x-Achse.

Zweckmäßigerweise ergibt der Verlauf der Messwerte bei dem erfindungsgemäßen Verfahren, zumindest annähernd, eine e-Funktion bzw. Exponentialfunktion. Anhand des Kurvenverlaufs der Messwerte können wiederum Charakteristika der Kognitionszeit der betreffenden Testperson berechnet bzw. abgeleitet werden.

Bei der kognitiven Erkennungschwelle S(Schwellwert) handelt es sich vorzugsweise um einen Schwierigkeitsgrad des kognitiven Erkennens, bei dem das Vermögen des kognitiven Erkennens durch die Testperson innerhalb des vorgegebenen Zeitfensters für die Reaktion auf den jeweiligen Stimulus gerade noch positiv ist. Beispielsweise kann die Testperson die kognitive Aufgabe gerade noch innerhalb der vorgegebenen Zeitspanne beantworten.

Zweckmäßigerweise werden die Messungen bzw. Verfahrensschritte zum Erhalt der Reaktionszeiten in statistisch ausreichender Zahl wiederholt, um statistisch aussagekräftige Messwerte zu erhalten.

Ganz besonders vorteilhaft ist das erfindungsgemäße Verfahren in Bezug auf die Erfassung der zerebralen Kognitionszeit bei visuellen Stimuli. Die Bestimmung der Kognitionszeit nach obiger Beschreibung bei unterschiedlichen Schwierigkeitsgraden und speziell bei der sensorischen Schwelle von visuellen Stimuli erfolgt für unterschiedliche Sehkanäle oder Sehfilter für die beidäugige Stereopsis. Gemäß der Erfindung handelt es sich bei den Stimuli um stereoskopische Testbilder mit variierender Stereodisparität. Hierdurch kann z. B. die stereoskopische Sehfähigkeit von Testpersonen hinsichtlich der stereoskopischen Schwelle überprüft und die zerebralen Kognitionszeiten von unterschiedlichen Stereoskopieschwierigkeitsgraden genau erfasst werden.

Vorzugsweise kann auch die Kognitionszeit von visuellen Stimuli für Bewegungssehen oder Farbsehen durch das erfindungsgemäße Verfahren bestimmt werden.

Vorzugsweise handelt es sich bei den Stimuli um Stimuli gleicher Art und/oder Kategorie.

Erfindungsgemäß werden die Stimuli in Bezug auf ihren Schwierigkeitsgrad der Testperson stochastisch bzw. in zufälliger Abfolge angeboten. Dies bedeutet, dass Stimuli mit sich zufällig änderndem Schwierigkeitsgrad im Rahmen des erfindungsgemäßen Verfahrens generiert werden. Hierdurch werden Anlerneffekte vermieden, durch die das Messergebnis verfälscht werden könnte.

Die vorliegende Erfindung betrifft des Weiteren eine Vorrichtung zur Erfassung der zerebralen Kognitionszeit einer Testperson gemäß dem Oberbegriff des Anspruchs 10. Die Vorrichtung ist dadurch gekennzeichnet, dass sie konfiguriert ist, um das Verfahren gemäß den Ansprüchen 1 bis 9 durchzuführen .

Zweckmäßigerweise umfasst die Vorrichtung einen Zufallsgenerator zur Erzeugung der Stimuli unterschiedlicher Schwierigkeitsgrade nach dem Zufallsgeneratorprinzip.

Die abgeleiteten Kognitionszeiten K(Schwellwert) oder K(x) eignen sich ganz besonders als Grundlage für eine Überwachung eines Therapie- und/oder Trainingsfortschritts bei der Therapie und/oder beim Trainieren der zerebralen Kognitionsfähigkeit.

Demzufolge kann die beanspruchte Vorrichtung auch Bestandteil eines Trainings- oder Therapiegerätes für die zerebrale Kognitionsfähigkeit sein.

### Beschreibung der Erfindung anhand von Ausführungsbeispielen

Zweckmäßige Ausgestaltungen der vorliegenden Erfindung werden anhand der Zeichnungsfiguren näher erläutert. Es zeigen:
- Fig. 1: eine stark vereinfachte Darstellung des Zusammenhangs zwischen dem Schwierigkeitsgrad S einer kognitiven Aufgabe und der Reaktionszeit R einer Testperson;
- Fig. 2: den Zusammenhang zwischen der korrekt erkannten Stereodisparität von unterschiedlichen Schwierigkeitsgraden und den korrespondierenden Reaktionszeiten dieser Stimuli; sowie
- Fig. 3: eine stark vereinfachte schematische Darstellung einer Einrichtung zur Durchführung des Verfahrens.

Das erfindungsgemäße Verfahren betrifft die Messung der Reaktionszeiten einer Testperson bei einer Mehrzahl von Stimuli mit unterschiedlichen Schwierigkeitsgraden hinsichtlich des Erkennens für einen individuellen Reiz. Das Verfahren umfasst folgende Verfahrensschritte:
(a) Bereitstellen eines Stimulus für einen individuellen Reiz als Aufgabe für die Testperson P zum Erkennen und Untersuchen, ob dieser Reiz von der Testperson P erkannt wird,
(b) Präsentation des Stimulus für eine vorgegebene Zeitdauer, so dass die Testperson P den Stimulus wahrnehmen kann, wobei die Präsentation des Stimulus unterschiedlich lange erfolgen kann
(c) motorische Reaktion der Testperson P auf den von ihr wahrgenommenen Stimulus innerhalb einer Reaktionszeit, wobei sich die Reaktionszeit zumindest auf einen kognitiven sowie motorischen Zeitanteil aufteilt,
(d) Erfassung der Reaktionszeit als Zeitdauer zwischen Beginn der Präsentation des Stimulus und Abschluss der motorischen Reaktion der Testperson P als Folge des Stimulus,
wobei die Verfahrensschritte (a) bis (d) fortlaufend mit Stimuli, vorzugsweise Stimuli gleicher Art oder Kategorie, mit variierendem kognitiven Schwierigkeitsgrad für die Testperson durchgeführt werden und hierbei eine Reihe von Messwerten der Reaktionszeit zu dem Stimulus mit unterschiedlichen kognitiven Schwierigkeitsgrad erzeugt wird, und zur Erfassung der zerebralen Kognitionszeit eine Korrektur der Messwerte der Reaktionszeit zu einer abgeleiteten Kognitionszeit K(x) vorgenommen wird, derart, dass die Messwerte der Reaktionszeit T um einen Reaktionszeit-Anteil T1 bereinigt werden, der sich aus dem asymptotischen Verlauf der Messwerte hin zu geringeren Schwierigkeitsgraden ergibt.

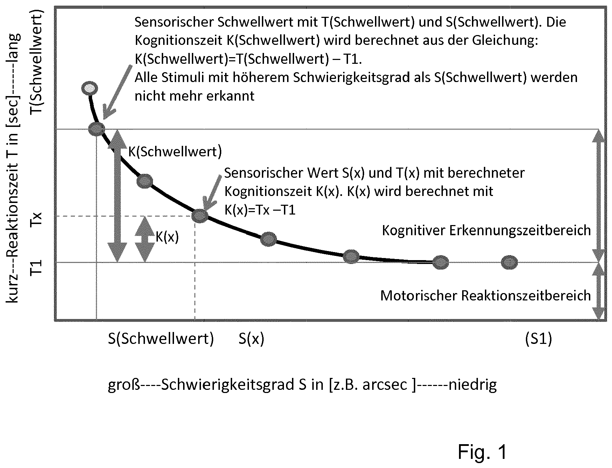

Wie aus Fig. 1 ersichtlich ergeben die Messpunkte eine von rechts nach links in Fig. 1 ansteigende, mit einer e-Funktion vergleichbare Kurve. Der Schwierigkeitsgrad S der kognitiven Aufgabe steigt auf der Abszisse von der rechten zur linken Seite in Fig. 1 an. Die Ordinate bildet die Reaktionszeit T (in sec), d.h. die Reaktionszeit der Testperson gemessen vom Beginn der Präsentation des Stimulus bis zum Abschluss der motorischen Reaktion der Testperson als Reaktion auf den Stimulus, beispielsweise bis zum Drücken einer Taste oder dergleichen. Der Schwierigkeitsgrad S(Schwellwert) definiert denjenigen Schwierigkeitsgrad, d.h. einen Stimulus mit einem bestimmten Schwierigkeitsgrad, der von der Testperson gerade noch erkannt werden kann. Jenseits der Schwierigkeitsgradschwelle S(Schwellwert) sind Reaktionen lediglich nur noch zufällig.

Anstatt einer Reaktion durch Tastendruck kann auch eine Gestensteuerung, z.B. mit Armen oder Beinen, vorgesehen sein, bei der Gesten als Reaktion auf die Stimuli erfasst werden.

Wie aus Fig. 1 deutlich wird, ist die Reaktionszeit T auf einen Stimulus die Summe aus Kognitionszeit (kognitiver Bereich) sowie motorischer Reaktionszeit (motorischer Bereich). Die Kognitionszeit ist abhängig vom Schwierigkeitsgrad S des Stimulus. Je schwieriger, d.h. komplexer der Stimulus, desto länger ist die Kognitionszeit K. Die motorische Reaktionszeit setzt nach Abschluss des Erkennungsprozesses ein und ist damit unabhängig vom Schwierigkeitsgrad des Stimulus.

Indem von der jeweils absolut gemessenen Reaktionszeit T der den motorischen Bereich kennzeichnende Reaktionszeit-Anteil T1 abgezogen wird, ergibt sich die abgeleitete Kognitionszeit K(x), wie dies für einen besonderen Schwierigkeitsgrad S(x) in Fig. 1 beispielshaft angegeben ist. Die abgeleitete Kognitionszeit K(x) stellt somit die eigentliche Zeit für die Erfassung und das Erkennen des Stimulus mit dem zugehörigen Schwierigkeitsgrad S dar.

Die Stimuli werden in unterschiedlichen Schwierigkeitsgraden S mehrmals nacheinander der Testperson präsentiert, wobei hierbei die Korrektheit der Antwort sowie die Reaktionszeit erfasst und dokumentiert werden. Um Anlerneffekte auszuschließen, werden die Stimuli unterschiedlicher Schwierigkeitsgrade S der Testperson stochastisch, d.h. per Zufallszuordnung präsentiert. Die jeweils korrespondierenden Reaktionszeiten für korrekte Antworten werden mittels eines Zeiterfassungsglieds gemessen. Die in Fig. 1 dargestellte Kurve ist stellvertretend für eine Vielzahl von Messpunkten bei Stimuli unterschiedlichen Schwierigkeitsgrades S.

Da die motorische Reaktionszeit T1 je nach Testperson große Unterschiede aufweisen kann, wird mit dem erfindungsgemäßen Verfahren nunmehr die Möglichkeit geschaffen, eine abgeleitete Kognitionszeit K(x) zu bilden, die für den individuellen Anwendungsfall ausschließlich die Leistungsfähigkeit des kognitiven Bereichs unter Ausschluss des motorischen Bereichs abbildet und damit als Referenzvariable und/oder Steuervariable für ein individuelles Diagnose- und/oder Trainingsverfahren eingesetzt werden kann.

Das Verfahren eignet sich ganz besonders zur Bestimmung der Kognitionszeit von visuellen Stimuli für die Stereopsis. Hierfür können entsprechende Stimuli mit variierenden Schwierigkeitsgraden gebildet und entsprechende Messwert-Kurven aufgezeichnet werden.

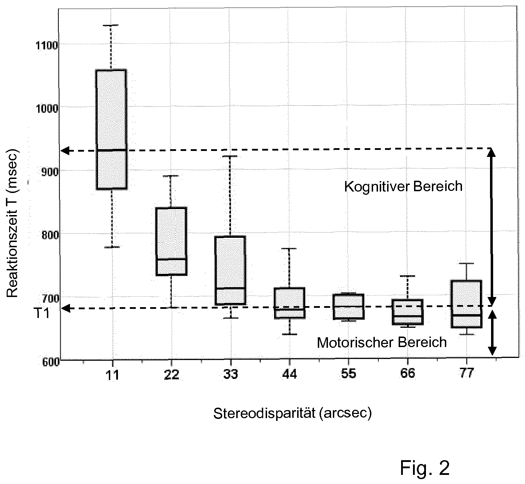

Die Darstellung in Fig. 2 zeigt eine entsprechende Kurve der Stereodisparität (S in arcsec) mit von rechts nach links steigendem Schwierigkeitsgrad über der Reaktionszeit T (in msec). Wie aus Fig. 2 deutlich wird, bilden die einzelnen Messwerte eine asymptotische Kurve näherungsweise nach Art einer e-Funktion bzw. Exponentialfunktion. Die asymptotische Kurve legt eine untere Reaktionszeit T1 fest, die die motorische Reaktion bei geringen Kognitions-Schwierigkeitsgraden der betreffenden Testperson wiederspiegelt. Somit müssen die betreffenden Messwerte der Reaktionszeiten T bei den unterschiedlichen Stimuli im konkreten Beispiel um den motorischen Reaktionszeit-Anteil von ca. 680 msec bereinigt werden, sodass die Messwerte ausschließlich Kognitionszeiten repräsentieren. Beispielsweise beträgt die Kognitionszeit K(x) bei einem Schwierigkeitsgrad der Stereodisparität von 11 arcsec (K(11arcsec) 260 msec. Hierbei handelt es sich um den sensorischen Schwellwert. Der Wert der Kognitionszeit bei diesem Schwellwert beträgt ca. 260 msec und ergibt sich aus einer Differenz der Reaktionszeit T(Schwellwert) von 940 msec und dem motorischen Reaktionszeit-Anteil T1 von 680msec.

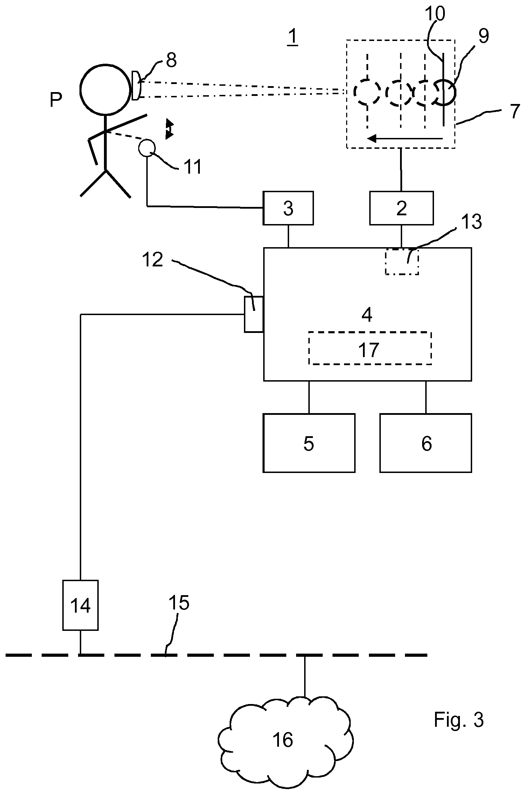

Fig. 3 zeigt als Beispiel eine Einrichtung zur Erfassung der zerebralen Kognitionszeit zum Erkennen und Untersuchen der zerebralen Kognitionszeit einer Testperson P. Die Einrichtung 1 umfasst ein Bilderzeugungsmodul 2, welches dazu dient, einen Stimulus 9, im vorliegenden Fall einen auf die Testperson P dreidimensional wirkenden Stimulus in Form von 4 Bällen, wobei ein Ball mit einer bestimmten Disparitätsdifferenz zu den anderen 3 Bällen dargestellt wird, in einem Monitor 7 bzw. Display 6 zu erzeugen. Der Stimulus 9 dient zur Feststellung der zerebralen Kognitionszeit von zum Beispiel stereoskopischen Bildern unterschiedlicher Schwierigkeitsgrade, hier mit unterschiedlichen Disparitäten. Hierzu wird der Stimulus 9, z.B. 4 Bälle, wobei ein Ball mit einer bestimmten Disparitätsdifferenz 10 zu den anderen 3 Bällen dargestellt wird, erzeugt. Die Testperson P kann zu diesem Zweck mit einer 3D-Brille 8 ausgestattet sein. Der Stimulus 9 mit 4 Bällen, wobei ein Ball einen Disparitätsunterschied aufweist, wird entsprechend Fig. 3 der Testperson P nacheinander mit verschiedenen Disparitätsdifferenzen und damit unterschiedlichen virtuellen räumlichen Ebenen 10 präsentiert. Wenn die Testperson P den Stimulus 9, d.h. den Ball mit dem Disparitätsunterschied korrekt erkennt und diese Frage richtig beantwortet (z.B. Tastendruck rechts oben, da Ball rechts oben näher an der Testperson erscheint, als die anderen 3 Bälle), wird von ihr eine manuelle Eingabe 11, z.B. ein Taster oder ein Touchpad, betätigt und von einer Zeiterfassungseinrichtung 3 die Zeit von der Präsentation des Stimulus 9 bis zur Betätigung der manuellen Eingabeeinrichtung 11 gemessen und einem Steuer- und Auswertemodul 4 zugeführt. Danach wird der Test mit dem dreidimensional wirkenden Stimulus 9 in Form von 4 Bällen, wobei nun ein anderer Ball in einer anderen Disparitätsdifferenz und damit in einer anderen optischen Ebene 10 dargestellt wird fortgesetzt. Das Steuer- und Auswertemodul 4 kann eine Ausgabe 5 sowie ein Display 6 zur Ausgabe bzw. zum Anzeigen der Messwerte aufweisen. Ferner umfasst das Steuer- und Auswertemodul 4 zweckmäßigerweise eine Speichereinrichtung 17. Die visuellen Stimuli 9 unterschiedlichen Schwierigkeitsgrads sind in Fig. 3 jeweils gestrichelt dargestellt. Beispielsweise kann hierbei ein Stimulus 9 in Form von 4 Bällen, wobei immer ein anderer Ball in einer anderen Disparitätsdifferenz und damit in einer anderen optischen Ebene als die anderen 3 Bälle 10 dargestellt wird, der Testperson P präsentiert werden. Der Ball in der anderen Disparitätsdifferenz als die anderen 3 Bälle erscheint in einer anderen optischen Ebene 10 als die anderen Bälle.

Zweckmäßigerweise umfasst das Steuer- und Auswertemodul 4 einen Zufallsgenerator 13, welcher dafür sorgt, dass die einzelnen Stimuli 9 unterschiedlichen Schwierigkeitsgrads S zufällig der Testperson P präsentiert werden, d.h. eine fortlaufende Präsentation mit allmählich steigendem Schwierigkeitsgrad somit ausgeschlossen wird, da andernfalls kognitive Lerneffekte das Messergebnis verfälschen können.

Die Einrichtung 1 ermöglicht es, entsprechende Kurvenverläufe, wie z.B. in Fig. 1 oder Fig. 2 dargestellt, zu erzeugen und daraus die abgeleitete Kognitionszeit K(x), die alleine den kognitiven Bereich der Reaktionszeit abbildet, zu generieren. Hierbei handelt es sich um die jeweils individuelle Kognitionszeit K(x) der betreffenden Testperson P, welche beispielsweise in einem internen Speicher 17 der Einrichtung 1 abgelegt werden kann.

Alternativ oder zusätzlich kann die abgeleitete Kognitionszeit K(x) auch über eine Datenausgabe 12, beispielsweise ein Interface, an andere Nutzer, z.B. an ein Modem 14, ausgegeben werden. Im Bedarfsfall können die Daten über das Modem 14, einem Netzwerk bzw. dem Internet 15 zugeführt und von dort z.B. in einer Computercloud 16 zentral abgelegt werden.

Die vorliegende Erfindung ermöglicht es, mit vergleichsweise einfachen Mitteln die Kognitionszeit von unterschiedlich schwierig zu detektierender Reizen einer Testperson ohne verfälschende motorische Reaktionszeitkomponente zu generieren und für Diagnose- und/oder Trainingsanwendungen zu nutzen. Infolgedessen kann die erfindungsgemäße Einrichtung auch Gegenstand eines Diagnose- und/oder Trainingsgeräts sein, mit dem die kognitive Leistungsfähigkeit in Zusammenhang mit der Verarbeitung von z.B. visuellen Reizen diagnostiziert und/oder sogar trainiert werden kann.

### BEZUGSZEICHENLISTE

- 1: Vorrichtung
- 2: Bilderzeugungsmodul
- 3: Zeiterfassungseinrichtung
- 4: Steuer- und Auswertemodul
- 5: Ausgabe
- 6: Display
- 7: Monitor
- 8: 3D-Brille
- 9: Stimulus
- 10: optische Ebene
- 11: manuelle Eingabe
- 12: Datenausgabe
- 13: Zufallsgenerator
- 14: Modem
- 15: Internet
- 16: Computercloud

- P: Testperson
- S: Schwierigkeitsgrad
- T: Reaktionszeit
- K(x): abgeleitete Kognitionszeit

## Patentansprüche

1. Verfahren zur Erfassung der zerebralen Kognitionszeit einer Testperson P mit folgenden Verfahrensschritten:
(a) Bereitstellen eines Stimulus für einen individuellen Reiz als Aufgabe für die Testperson P zum Erkennen und Untersuchen der Kognitionszeit der Testperson P,
(b) Präsentation des Stimulus für eine vorgegebene Zeitdauer, so dass die Testperson P den Stimulus wahrnehmen kann,
(c) Reaktion der Testperson P auf den von ihr wahrgenommenen Stimulus innerhalb einer Reaktionszeit T, wobei sich die Reaktionszeit zumindest auf einen kognitiven sowie motorischen Zeitanteil aufteilt,
(d) Bestimmung der Reaktionszeit T als Zeitdauer zwischen der Präsentation des Stimulus und der motorischen Reaktion der Testperson P als Folge des Stimulus,
wobei die Verfahrensschritte (a) bis (d) fortlaufend mit Stimuli mit variierendem kognitiven Schwierigkeitsgrad S für die Testperson durchgeführt werden,
wobei zur Erfassung der zerebralen Kognitionszeit eine Korrektur der Messwerte der Reaktionszeit zu einer abgeleiteten Kognitionszeit K(x) vorgenommen wird, derart, dass die Messwerte der Reaktionszeit T um einen motorischen Reaktionszeit-Anteil T1 bereinigt werden, der sich aus dem asymptotischen Verlauf der individuellen Messwertkurve hin zu geringeren Schwierigkeitsgraden S ergibt,
und wobei eine Referenzvariable und/oder Steuervariable auf der Basis der abgeleiteten personen-individuellen Kognitionszeit K(Schwellwert) oder (K(x) generiert wird,
**dadurch gekennzeichnet, dass**
es sich bei den visuellen Stimuli um Stimuli für die Stereopsis in Form von stereoskopischen Testbildern mit variierender Stereodisparität handelt,
eine Reihe von Messwerten der Reaktionszeit zu dem jeweiligen kognitiven Schwierigkeitsgrad in Form einer individuellen asymptotischen Messwertkurve aus Reaktionszeiten für unterschiedliche Schwierigkeitsgrade S in arcsec erzeugt wird,
die Stimuli in Bezug auf ihren Schwierigkeitsgrad der Testperson stochastisch oder in zufälliger Abfolge angeboten werden und
der Kurvenverlauf der Messwerte und/oder die Kurvenfläche als Charakteristikum der kognitiven Leistungsfähigkeit der Testperson verwendet wird.

2. Verfahren nach Anspruch 1, **dadurch gekennzeichnet, dass**
als motorischer Reaktionszeit-Anteil T1 eine Reaktionszeit festgelegt wird, die die Reaktionszeit für Stimuli mit geringem kognitiven Schwierigkeitsgrad S1 anhand der Reihe von Messwerten der Reaktionszeit T repräsentiert oder zumindest mit dieser korreliert.

3. Verfahren nach Anspruch 1 oder 2, **dadurch gekennzeichnet, dass** eine Reaktionszeit T(x) festgelegt wird und
eine abgeleitete Kognitionszeit K(x) als Differenz aus der Reaktionszeit T(x) und dem motorischen Reaktionszeit-Anteil (T1) gebildet wird.

4. Verfahren nach mindestens einem der vorhergehenden Ansprüche, **dadurch gekennzeichnet, dass** eine Schwellwert-Reaktionszeit T(Schwellwert) festgelegt wird, die die Reaktionszeit bei der kognitiven Erkennensschwelle S(Schwellwert) anhand der Reihe von Messwerten der Reaktionszeit T bei unterschiedlichen Stimuli repräsentiert, und
eine abgeleitete Kognitionszeit K(Schwellwert) als Differenz aus der Reaktionszeit T(Schwellwert) und dem motorischen Reaktionszeit-Anteil (T1) gebildet wird.

5. Verfahren nach mindestens einem der vorhergehenden Ansprüche, **dadurch gekennzeichnet, dass** die abgeleitete Kognitionszeit K(Schwellwert) oder K(x) als Referenzvariable und/oder Steuervariable für ein Diagnose- und/oder Trainingsverfahren zur Diagnose bzw. zum Training der kognitiven Leistungsfähigkeit verwendet wird.

6. Verfahren nach mindestens einem der vorhergehenden Ansprüche, **dadurch gekennzeichnet, dass** es sich bei der kognitiven Schwierigkeitsschwelle S(Schwellwert) um den höchsten Schwierigkeitsgrad handelt, den die Testperson P noch erkennt.

7. Verfahren nach mindestens einem der vorhergehenden Ansprüche, **dadurch gekennzeichnet, dass** es sich bei der Reaktionszeit T1 um einen Zustand oder Streubereich der Reaktionszeit T handelt, bei dem die Reaktionszeit T bei sich abnehmenden Schwierigkeitsgrad konstant bleibt oder zumindest eine bestimmte Reaktionszeit-Bandbreite nicht verlässt.

8. Verfahren nach mindestens einem der vorhergehenden Ansprüche, **dadurch gekennzeichnet, dass** zum Erhalt statistisch aussagekräftiger Messwerte die Reaktionszeitmenge bei den verschiedenen Schwierigkeitsgraden in ausreichender Anzahl wiederholt werden.

9. Verfahren nach Anspruch 1, **dadurch gekennzeichnet, dass** es sich bei den optischen Stimuli für Stereopsis um fixe oder sich bewegende 3-dimensionale Objektbilder handelt, die in unterschiedlichen (auch virtuellen) Entfernungen zur Testperson P erscheinen.

10. Vorrichtung (1) zur Erfassung der zerebralen Kognitionszeit einer Testperson P mit
einer Einrichtung zur Erzeugung und Präsentation eines Stimulus (9) als individuellen Reiz als Aufgabe für die Testperson P zum Erkennen und Untersuchen der kognitiven Erkennungsfähigkeit der Testperson P,
einer Zeiterfassungseinrichtung (3) zur Erfassung der Zeit von der Präsentation des Stimulus bis die Testperson P auf die Präsentation des Stimulus körpermotorisch reagiert hat,
einer von der Testperson betätigbaren, vorzugsweise manuell oder akustisch betätigbaren Eingabevorrichtung (11) zur Auslösung der Zeiterfassungseinrichtung (3),
einem Steuerungs- und Auswertemodul (4), sowie einem Zufallsgenerator (13)
**dadurch gekennzeichnet, dass** die Vorrichtung (1) konfiguriert ist, um das Verfahren gemäß den Ansprüchen 1 bis 9 durchzuführen.

11. Vorrichtung nach Anspruch 10, **dadurch gekennzeichnet, dass** die Vorrichtung Bestandteil eines Trainings- oder Therapiegerätes für die zerebrale Kognitionsfähigkeit ist.

## Claims

1. Method for capturing the cerebral recognition time of a subject P, including the following method steps:
(a) provision of a stimulus for an individual stimulation as a problem for the subject P for the purposes of recognizing and examining the recognition time of the subject P,
(b) presentation of the stimulus for a predetermined period of time such that the subject P can perceive the stimulus,
(c) reaction of the subject P to the stimulus perceived by them within a reaction time T, the reaction time being divided into at least a cognitive time component and a motoric time component,
(d) determination of the reaction time T as a period of time between the presentation of the stimulus and the motoric reaction of the subject P as a consequence of this stimulus,
wherein method steps (a) to (d) are carried out continuously with stimuli of varying cognitive difficulty S for the subject,
wherein the measurement values of the reaction time are corrected to form a derived cognition time K(x) for the purposes of capturing the cerebral recognition time in such a way that a motoric reaction time component T1, which emerges from the asymptotic profile of the individual measurement value curve at lower difficulties S, is removed from the measurement values of the reaction time T,
and wherein a reference variable and/or control variable is generated on the basis of the derived cognition time K (threshold) or K(x) that is individual to a person,
**characterized in that**
the visual stimuli are stimuli for stereopsis in the form of stereoscopic test images with a varying stereo disparity,
a sequence of measurement values of the reaction time for the respective cognitive difficulty is produced in the form of an individual asymptotic measurement value curve from reaction times for different difficulties S in arcsec,
the stimuli are offered stochastically or in a random order in relation to their difficulty to the subject and
the curve profile of the measurement values and/or the area of the curve is used as a characteristic of the cognitive power of the subject.

2. Method according to Claim 1, **characterized in that**
a reaction time is set as motoric reaction time component T1, said reaction time representing the reaction time for stimuli with a low cognitive difficulty S1 on the basis of the sequence of measurement values of the reaction time T or said reaction time at least correlating therewith.

3. Method according to Claim 1 or 2, **characterized in that** a reaction time T(x) is set and
a derived cognition time K(x) is formed as the difference between the reaction time T(x) and the motoric reaction time component (T1).

4. Method according to at least one of the preceding claims, **characterized in that** a threshold reaction time T(threshold) is set, the threshold reaction time representing the reaction time at the cognitive recognition threshold S(threshold) on the basis of the sequence of measurement values of the reaction time T in the case of different stimuli, and
a derived cognition time K(threshold) is formed as the difference between the reaction time T(threshold) and the motoric reaction time component (T1).

5. Method according to at least one of the preceding claims, **characterized in that** the derived cognition time K(threshold) or K(x) is used as reference variable and/or control variable for a diagnostic and/or training method for diagnosing or training the cognitive power.

6. Method according to at least one of the preceding claims, **characterized in that** the cognitive difficulty threshold S(threshold) is the greatest difficulty still identified by the subject P.

7. Method according to at least one of the preceding claims, **characterized in that** the reaction time T1 is a state or range of variation of the reaction time T, during which the reaction time T remains constant in the case of reducing difficulty or at least does not depart from a certain reaction time bandwidth.

8. Method according to at least one of the preceding claims, **characterized in that** the reaction time quantity is repeated in sufficient numbers at the various difficulties for the purposes of obtaining statistically meaningful measurement values.

9. Method according to Claim 1, **characterized in that** the optical stimuli for stereopsis are fixed or moving 3-dimensional object images which appear at different distances (also virtual distances) from the subject P.

10. Apparatus (1) for capturing the cerebral cognition time of a subject P, comprising
a device for producing and presenting a stimulus (9) as an individual stimulation as a problem for the subject P for the purposes of recognizing and examining the cognitive recognition capability of the subject P,
a time capturing device (3) for capturing the time elapsed between the presentation of the stimulus and the subject P having reacted in body-motoric fashion to the presentation of the stimulus,
an input apparatus (11) for triggering the time capturing device (3), said input apparatus being actuatable by the subject, preferably actuatable in manual or acoustic fashion,
a control and evaluation module (4) and
a random number generator (13)
**characterized in that** the apparatus (1) is configured to carry out the method according to Claims 1 to 9.

11. Apparatus according to Claim 10, **characterized in that** the apparatus is a constituent part of a training or therapy appliance for cerebral cognitive ability.

## Revendications

1. Procédé de détection du temps de réponse cognitif cérébral d'un sujet de test P, comprenant les étapes suivantes :
(a) fourniture d'un stimulus pour une excitation individuelle en tant que tâche pour le sujet de test P en vue de reconnaître et d'analyser le temps de réponse cognitif du sujet de test P,
(b) présentation du stimulus pendant une durée prédéfinie, de sorte que le sujet de test P puisse percevoir le stimulus,
(c) réaction du sujet de test P au stimulus qu'il a perçu dans un temps de réaction T, le temps de réaction se divisant au moins en une part de temps cognitive et une part de temps motrice,
(d) définition du temps de réaction T en tant que durée entre la présentation du stimulus et la réaction motrice du sujet de test P en tant que conséquence du stimulus,
les étapes de procédé (a) à (d) étant exécutées continuellement avec des stimuli ayant un degré de difficulté S cognitif variable pour le sujet de test P, la détection du temps de réponse cognitif cérébral étant effectuée en réalisant une correction des valeurs mesurées du temps de réaction par un temps de réponse cognitif dérivé K(x) de telle sorte que les valeurs mesurées du temps de réaction T sont débarrassées d'une part de temps de réaction motrice T1 qui résulte du tracé asymptotique de la courbe de mesure individuelle vers les degrés de difficulté S plus faibles,
et une variable de référence et/ou une variable de commande étant générées sur la base du temps de réponse cognitif individuel à la personne dérivé K (valeur de seuil) ou K(x),
**caractérisé en ce que**
les stimuli visuels étant des stimuli pour la stéréopsie sous la forme d'images de test stéréoscopiques ayant une disparité stéréoscopique variable,
une série de valeurs mesurées du temps de réaction associé au degré de difficulté cognitif respectif est générée sous la forme d'une courbe de mesure asymptotique individuelle composée des temps de réaction pour différents degrés de difficulté S en arcsec,
les stimuli, en référence à leur degré de difficulté, sont proposés au sujet de test de manière stochastique ou dans un ordre aléatoire et
le tracé de la courbe des valeurs mesurées et/ou la surface de la courbe sont utilisés comme caractéristique de l'aptitude cognitive du sujet de test.

2. Procédé selon la revendication 1, **caractérisé en ce que** la part de temps de réaction motrice T1 définie est un temps de réaction qui représente le temps de réaction pour les stimuli ayant un faible degré de difficulté SI cognitif à l'aide de la série de valeurs mesurées du temps de réaction T ou qui est au moins corrélé avec celui-ci.

3. Procédé selon la revendication 1 ou 2, **caractérisé en ce qu'**un temps de réaction T(x) est défini et un temps de réponse cognitif dérivé K(x) est formé en tant que différence entre le temps de réaction T(x) et la part de temps de réaction motrice T1.

4. Procédé selon au moins l'une des revendications précédentes, **caractérisé en ce qu'**un temps de réaction à valeur de seuil T(valeur de seuil) est défini, lequel représente le temps de réaction lors du seuil de reconnaissance cognitif S(valeur de seuil) à l'aide de la série de valeurs mesurées du temps de réaction T avec différents stimuli et
un temps de réponse cognitif dérivé K(valeur de seuil) est formé en tant que différence entre le temps de réaction T(valeur de seuil) et la part de temps de réaction motrice T1.

5. Procédé selon au moins l'une des revendications précédentes, **caractérisé en ce que** le temps de réponse cognitif dérivé K(valeur de seuil) ou K(x) est utilisé comme variable de référence et/ou variable de commande pour un procédé de diagnostic et/ou d'entraînement au diagnostic ou en vue de l'entraînement de l'aptitude cognitive.

6. Procédé selon au moins l'une des revendications précédentes, **caractérisé en ce que** le seuil de difficulté cognitive S(valeur de seuil) est le degré de difficulté le plus élevé que le sujet de test P reconnaît encore.

7. Procédé selon au moins l'une des revendications précédentes, **caractérisé en ce que** le temps de réaction T1 est un état ou une plage de dispersion du temps de réaction T dans lequel/laquelle le temps de réaction T reste constant en présence d'un degré de difficulté décroissant ou au moins ne quitte pas une largeur de bande de réaction définie.

8. Procédé selon au moins l'une des revendications précédentes, **caractérisé en ce qu'**en vue d'obtenir des valeurs mesurées statistiquement significatives, les quantités de temps de réaction sont répétées dans un nombre suffisant aux différents degrés de difficulté.

9. Procédé selon la revendication 1, **caractérisé en ce que** les stimuli optiques pour la stéréopsie sont des images d'objet tridimensionnelles fixes ou mobiles qui apparaissent à des distances (également virtuelles) différentes du sujet de test P.

10. Arrangement (1) de détection du temps de réponse cognitif cérébral d'un sujet de test P, comprenant
un dispositif destiné à générer et à présenter un stimulus (9) en tant qu'excitation individuelle en tant que tâche pour le sujet de test P en vue de reconnaître et d'analyser l'aptitude de reconnaissance cognitive du sujet de test P,
un dispositif de détection du temps (3) destiné à détecter le temps entre la présentation du stimulus et la réaction motrice corporelle du sujet de test P à la présentation du stimulus,
un arrangement de saisie (11) pouvant être actionné par le sujet de test, de préférence pouvant être actionné manuellement ou de manière sonore, pour déclencher le dispositif de détection du temps (3),
un module de commande et d'interprétation (4), ainsi que
un générateur aléatoire (13)
**caractérisé en ce que** l'arrangement (1) est configuré pour mettre en oeuvre le procédé selon les revendications 1 à 9.

11. Arrangement selon la revendication 10, **caractérisé en ce que** l'arrangement fait partie d'un appareil d'entraînement ou thérapeutique pour l'aptitude cognitive cérébrale.
